# EUROPEAN PATENT APPLICATION

(11) **EP 1 557 171 A1**
(43) Date of publication of application: **27.07.2005**
(21) Application number: 03770855.9
(22) Date of filing: 30.10.2003
(51) Int. Cl.: A61K 35/12, A61K 35/36, A61P 25/04, A61P 29/00

(54) **RABBIT SKIN COMPRISING BIOLOGICAL ACTIVE SUBSTANCE AND ITS USE**

(30) Priority: 31.10.2002 CN 02145975
(71) Applicant: VanWorld Pharmaceutical (Rugao) Company Limited, Jiangsu 226500 (CN)
(72) Inventor: CHEUNG, Wing, S., Jiangsu 226500 (CN)
(74) Representative: Behnisch, Werner, Dr.
(86) International application number: PCT/CN2003/000923
(87) International publication number: WO 2004/060381

(57) **Abstract**

The present invention relates to a rabbit skin containing biologically active substances. The rabbit skin is obtained by the process including vaccinating rabbit skin tissues with vaccinia virus, feeding rabbit vaccinated with vaccinia virus, killing the rabbit when its skin tissues is inflamed enough, and peeling the rabbit. The rabbit skin of the present invention can be used for preparing drugs and health foods.

## Description

### FIELD OF THE INVENTION

The present invention relates to a rabbit skin containing biologically active substances and its use.

### BACKGROUND OF THE INVENTION

It was reported that the extracts from inflammatory rabbit skin tissues vaccinated with vaccinia virus can be used for the treatment of allergic disease and have the analgesic effect. There has not been any method to prepare rabbit skin that contains strongly active and high yield biologically active substances.

### DETAILED DESCRIPTION OF THE INVENTION

### Aims

The objective of the present invention is to provide a rabbit skin which is rich in biologically highly active substances that can be used for preparing drugs and health foods.

### Project

As a result of many years of hard work, the inventors of the present invention have reached this aim.

The rabbit skin of the present invention is obtained by the process including vaccinating rabbit (*Oryctolagus cuniculus*) skin tissues with vaccinia virus, feeding the rabbit vaccinated with vaccinia virus, killing the rabbit when its skin tissues are inflamed enough, and peeling the rabbit.

Vaccinia virus has been used widely since the 20th century. All kinds of vaccinia virus can be used to prepare the rabbit skin of the present invention, such as Lister strain, Ikeda strain, Dairen strain, EM-63 strain, Temple of Heaven strain, LMC strain, Tashkent strain, Williamsport strain, and New York City Board of Health strain. The preferred strains are Lister strain, Ikeda strain, Dairen strain, EM-63 strain, the most preferred strain is Lister strain. All these vaccinia virus strains can be purchased from the market. The vaccinia virus strains used in the present invention can be a purchased strain or a strain obtained from a subculture with rabbit.

The preferred vaccination method is subcutaneous vaccination, said vaccinating of rabbit skin tissues with vaccinia virus is effected by injecting subcutaneously 0.1~0.4 ml solution containing 10⁶~10⁹ viruses/ml at each site, 100 to 250 sites per rabbit weighing 1.5~3 Kg.

The rabbit used in preparing the rabbit skin of the present invention can be all kinds of rabbits, such as Japanese white rabbit, New Zealand white rabbit, Chinese rabbit, Blue-violet rabbit, Silver Fox rabbit, Viennese rabbit, Long hair rabbit, Himalayan albio rabbit, Pex, Belgian Hare rabbit, Lop, California rabbit, Chekered Giant, Denmark white rabbit, West Germany long hair rabbit, the preferred rabbits are Japanese white rabbit, New Zealand white Rabbit, Chinese rabbit, Blue-violet rabbit, the most preferred rabbit is Japanese white rabbit.

The killing of the rabbit when its skin tissues are inflamed enough is effected when the rabbit skin inflammatory tissue shows visible blains accompanied with changing colour from redness to mauveness and becomes thick, and its subcuticle and hip become swollen. The preferred method to kill the rabbit is cervical vertebrae dislocation.

### Effect

The rabbit skin of the present invention has 0.5 iu/g SART activity or more, and it also possesses the kallikrein-protease inhibition activity.

By extracting with organic solvent, processing with acid, processing with alkali, absorbing, eluting and concentrating, biologically active preparations being rich in amino acids and nucleic acids can be prepared from the rabbit skin. Said amino acids include glutamic acid, glycine, alanine, valine, isoleucine, leucine, tyrosine, phenylalanine, lysine, histidine, aspartic acid, threonine and serine. Said nucleic acids include urocanic acid, uracil, hypoxanthine, xanthine and thymine.

A drug can be prepared by combining the biologically active preparations of the present invention with pharmaceutical acceptable adjuvants. This drug can have various forms of preparations used clinically, including injection and tablet, the preferred form is the injection. For injections, the adjuvants can be for example injectable distilled water, normal saline, injectable vegetable oil, glucose injection, propylene glycol, polyethylene glycol, or it may be all kinds of stabilizers and emulsifiers. For tablets, capsules and granules, the adjuvants can be excipients such as starch, lactose, mannitol; binders such as crystalline cellulose, arabic gum, corn starch, glutin, polyethylene, polyvinyl alcohol, polyvinyl-pyrrolidone; disintegrants such as carboxyl methyl cellulose, poly-ethylene glycol, potato starch; lubricants such as talcum powder, magnesium stearic acid; moistening agents such as glycerol. For ointments, the adjuvants can be fat oil, paraffin, wool fat, vaseline, glycol, glycerol.

The pharmacological and clinic experiments showed that the drugs prepared from the rabbit skin of the present invention have analgesic effect against all kinds of symptomatic neuralgia, lambago, cholecystagia, angina, arterial embolism pains, acute pains from wound, burn and scald, pains in surgery or post-surgery, peptic ulcer pain, dysmenorrhea, labor pains posterior to childbirth, headache, pains induced by various tumor and so on.

The study showed that the drugs prepared from the rabbit skin of the present invention can effectively promote activation of macrophage, significantly inhibit 48-hour homologous PCA reaction induced by antibody of IgE in the model of type I allergic reaction in mice, inhibit the activity of anti-complement in type II allergic reaction. The effects have linear correlation with the doses. So the drugs have effects on inhibiting inflammatory reaction correlated with immunity and improving immunity function.

Moreover, the drugs prepared from the rabbit skin of the present invention have anti-allergic, anti-ulcer and sedative effects and so on.

After a continuous 28-day intraperitoneal administration of the drugs prepared from the rabbit skin of the present invention in rats, no rats died and no changes induced by the drugs existed in examinations of urine, eye, blood biochemistry, pathology and anatomy. Therefore, the analgesic drugs of the present invention have little toxic effects.

Health food can be prepared by combining the biologically active preparations of the present invention with edible additives and nutritious substances, said edible additives and nutritious substances include all kinds of vitamins and flavoring agents and so on. These kinds of health food have effects on improving immunity, alleviating pains, anti-allergy and anti-stress and so on.

The method of determination of SART (Specific Alteration of Rhythm in Environmental Temperature) activity is well known in the art (Folia pharmacol. japon. 71:211~220, 1975).

The kallikrein-protease inhibition activity referred in this description is determined as follows:

The rabbit skin was cut in pieces of 1 cm², 4 times (w/w) of 3% phenol aqueous solution was added, the mixture then was placed at 4°C for 72h, centrifuged after the liquid changed into an emulsion. The supernatant was filtrated to collect a brown solution A. The brown solution A was boiled for 40 min in a water bath after pH was adjusted to 5.0 by 1M HCl, cooled to 28°C promptly, centrifuged and filtrated to collect solution B. The solution B was boiled for 40 min in a water bath after pH of filtrate was adjusted to 9.2 by 1M NaOH, cooled to 28°C promptly, and filtrated to collect solution C. pH of the solution C was adjusted to 4.5 by 1M HCl, activated charcoal was added at 30°C, stirred continuously for 4h, stopped stirring, left it for 30min, removed the supernatant, filtrated under nitrogen atmosphere. Then the activated charcoal was dipped in injectable water and washed, filtrated and discarded the filtrate to collect the activated charcoal, reserved the activated charcoal. The activated charcoal was then put into injectable water, adjusted pH to 11.0 by 1M NaOH, stirred continuously for 4h, filtrated with a 0.45-µm Millipore filter under nitrogen atmosphere, washed the activated charcoal by injectable water to collect solution D. After pH of the solution D was adjusted to 6.0 by 1M HCl, the vessel was sealed, heated up and kept the temperature at 121°C for 20min, and then cooled down to under 40°C to collect solution E. The solution E was placed into a decompression distillator, replaced the air with nitrogen in the decompression distillator, distilled at 60°C under decompression, filtrated to collect a solution containing biologically active substances, whose SART activity was determined. The SART activity of the solution was adjusted to 1.2iu/ml by evaporating, concentrating and diluting with distilled water. 10ml of this solution was desalted at 10 µS/cm of final conductance, dried under decompression condition, into which 1.5 ml of 0.25 M NaCl solution was added to obtain a test solution. 0.2 ml of 0.25M NaCl solution was regarded as the control solution and treated with parallel process with the 0.2 ml test solution. 0.5 ml human plasma were added respectively to both solutions, placed at freezing point for 5 min, added 0.25 ml of suspension of argilla, placed at freezing point for 20 min, and filtrated. 0.1 ml of the filtrate was mixed with 0.2 ml of 0.1 M Tris-HCl buffer and 0.1 ml of basic solution. Effected reaction for 20 min at 30°C, and stopped the reaction by adding 0.8 ml of 1% citric acid. The absorbance A of the test solution was determined at 405 nm as the absorbance of control solution was initialized as 0.4. If A was less than 0.4, the rabbit skin from which the test solution was prepared was regarded as possessing the kallikrein-protease inhibition activity.

### EXAMPLES

The present invention will be illustrated with the following non-limiting examples.

### Example 1

### Preparation of rabbit skin

The dry variola vaccine of vaccinia virus Lister strain was dissolved by PBS(-) (NaCl 80 g, KCl 2 g, NaH₂PO₄ 11.5 g, KH₂PO₄·2H₂O 2 g, adding injectable H₂O to 10 L) and well shaken. 0.4 ml of this solution was injected into the central inner lamina of the testicle of a Japanese white rabbit. Dislocating its cervical vertebra on the fourth day. Cut the scrotum, removed connective tissue in the testicle. The testicle was placed in a vessel full of ice, preserved at -80°C refrigerator. The testicle was taken from the refrigerator, softened for 1 h, grinded at 4°C, mixed in 1:1 with EAGLES' cultural medium (Eagle's powder 9.4 g, 10% NaHCO₃ 12.5~22.0 ml, glutamine 10 ml, injectable H₂O 1 L), packed, frozen at -80°C in refrigerator for 1 h, thawed at 37°C in a water bath, centrifuged with 3500 rpm at 4°C for 20 min, packed by 10 ml. The subculture antigen was preserved at -80°C in a refrigerator.

The virus solution of subculture antigen was taken from the -80°C refrigerator, thawed at 30°C in an incubator. 5 ml of the virus solution was added into 500 ml of PBS(-) by a 10-ml syringe, well shaken and diluted to an injection of 10⁹ virus/ml. A Japanese white rabbit weighing 3 kg with its back shaved, was sterilized with 75% ethanol, injected subcutaneously with the virus injection 0.4 ml per site in 200 sites with no leaking, no injecting without the virus injection and no puncturing throughout the skin. The rabbit injected was fed for 4 days. When inflammatory tissue showed that the skin surface had visible blains accompanied with changing colour from redness to mauveness and the skin became thick, and subcuticle and hip became swollen, the rabbit was killed by cervical vertebra dislocation and peeled in 15 min. The skin of the rabbit was packed in a plastic bag, preserved at -18°C in a refrigerator prior to use. The rabbit skin weighed 349 g, its SART activity was 0.85 iu/g, and its absorbance was 0.07. The results indicated that it possessed the kallikrein-protease inhibition activity.

### Example 2

### Preparation of rabbit skin

The vaccinia virus Ikeda strain and New Zealand white rabbit were used to prepare the subculture antigen according to example 1.

The virus solution of subculture antigen was taken from the -80°C refrigerator, thawed at 30°C in an incubator. 5 ml of the virus solution was added into 500 ml of PBS(-) by a 10-ml syringe, well shaken and diluted to an injection of 10⁹ virus/ml. A New Zealand white rabbit weighing 2.75 kg with its back shaved, was sterilized with 75% ethanol, injected subcutaneously with the virus injection 0.3 ml per site in 250 sites with no leaking, no injecting without the virus injection and no puncturing throughout the skin. The injected rabbit was fed for 3 days. When the inflammatory tissue showed that the skin surface had visible blains accompanied with changing colour from redness to mauveness and the skin became thick, and subcuticle and hip became swollen, the rabbit was killed by cervical vertebra dislocation and peeled in 15 min. The skin of the rabbit was packed in a plastic bag, preserved at -18°C in a refrigerator prior to use. The skin of the rabbit weighed 302 g, its SART activity was 0.60 iu/g, and its absorbance was 0.1. The results indicated that it possessed the kallikrein-protease inhibition activity.

### Example 3

### Preparation of rabbit skin

The vaccinia virus Dairen strain and Chinese rabbit were used to prepare the subculture antigen according to example 1.

The virus solution of the subculture antigen was taken from the -80°C refrigerator, thawed at 30°C in incubator. 5 ml of the virus solution was added into 500 ml of PBS(-) by a 10-ml syringe, well shaken and diluted to an injection of 10⁶ virus/ml. A Chinese rabbit weighing 1.5 kg with its back shaved, was sterilized with 75 % ethanol, injected subcutaneously with the virus injection 0.1 ml per site in 250 sites with no leaking, no injecting without the virus injection and no puncturing throughout skin. The injected rabbit was fed for 3 days. When the inflammatory tissue showed that skin surface had visible blains accompanied with changing colour from redness to mauveness and skin became thick, and subcuticle and hip became swollen, the rabbit was killed by cervical vertebra dislocation and peeled in 15 min. The skin of the rabbit was packed in a plastic bag, preserved at -18°C in a refrigerator prior to use. The skin of the rabbit weighed 176 g, its SART activity was 0.50 iu/g, and its absorbance was 0.15. The results indicated that it possessed the kallikrein-protease inhibition activity.

### Example 4

### Preparation of rabbit skin

The vaccinia virus EM-63 strain and Blue-violet rabbit were used to prepare the subculture antigen according to example 1.

The virus solution of the subculture antigen was taken from the -80°C refrigerator, thawed at 30°C in an incubator. 5 ml of the virus solution was added into 500 ml of PBS(-) by a 10-ml syringe, well shaken and diluted to an injection of 10⁷ virus/ml. A Blue-violet rabbit weighing 2 kg with its back shaved, was sterilized with 75% ethanol, injected subcutaneously with the virus injection 0.2 ml per site in 100 sites with no leaking, no injecting without the virus injection and no puncturing throughout skin. The injected rabbit was fed for 3 days. When inflammatory tissue showed that the skin surface had visible blains accompanied with changing colour from redness to mauveness and the skin became thick, and subcuticle and hip became swollen, the rabbit was killed by cervical vertebra dislocation and peeled in 15 min. The skin of the rabbit was packed in a plastic bag, preserved at -18°C in a refrigerator prior to use. The skin of rabbit weighed 230 g, its SART activity was 0.55 iu/g, and its absorbance was 0.12. The results indicated that it possessed the kallikrein-protease inhibition activity.

### Example 5

### Preparation of rabbit skin

The vaccinia virus Lister strain and New Zealand white rabbit were used to prepare the subculture antigen according to example 1.

The virus solution of the subculture antigen was taken from the -80□ refrigerator, thawed at 30°C in an incubator. 5 ml of the virus solution was added into 500 ml of PBS(-) by a 10-ml syringe, well shaken and diluted to an injection of 10⁹ virus/ml. A New Zealand white rabbit weighing 2.75 kg with its back shaved, was sterilized with 75% ethanol, injected subcutaneously with the virus injection 0.3 ml per site in 200 sites with no leaking, no injecting without the virus injection and no puncturing throughout skin. The injected rabbit was fed for 3 days. When inflammatory tissue showed that the skin surface had visible blains accompanied with changing colour from redness to mauveness and the skin became thick, and subcuticle and hip became swollen, the rabbit was killed by cervical vertebra dislocation and peeled in 15 min. The skin of the rabbit was packed in a plastic bag, preserved at - 18°C in a refrigerator prior to use. The skin of the rabbit weighed 310 g, its SART activity was 0.79 iu/g, and its absorbance was 0.09. The results indicated that it possessed the kallikrein-protease inhibition activity.

### Example 6

### Preparation of rabbit skin

The vaccinia virus Lister strain and Chinese rabbit were used to prepare the subculture antigen according to example 1.

The virus solution of the subculture antigen was taken from the -80°C refrigerator, thawed at 30°C in an incubator. 5 ml of the virus solution was added into 500 ml of PBS(-) by a 10-ml syringe, well shaken and diluted to an injection of 10⁶ virus/ml. A Chinese rabbit weighing 1.5 kg with its back shaved, was sterilized with 75% ethanol, injected subcutaneously with the virus injection 0.1 ml per site in 250 sites with no leaking, no injecting without the virus injection and no puncturing throughout skin. The injected rabbit was fed for 3 days. When inflammatory tissue showed that the skin surface had visible blains accompanied with changing colour from redness to mauveness and skin became thick, and subcuticle and hip became swollen, the rabbit was killed by cervical vertebra dislocation and peeled in 15 min. The skin of the rabbit was packed in a plastic bag, preserved at -18°C in a refrigerator prior to use. The skin of the rabbit weighed 185 g, its SART activity was 0.71 iu/g, and its absorbance was 0.11. The results indicated that it possessed the kallikrein-protease inhibition activity.

### Example 7

### Preparation of rabbit skin

The vaccinia virus Lister strain and Blue-violet rabbit were used to prepare the subculture antigen according to example 1.

The virus solution of the subculture antigen was taken from the -80°C refrigerator, thawed at 30°C in an incubator. 5 ml of the virus solution was added into 500 ml of PBS(-) by a 10-ml syringe, well shaken and diluted to an injection of 10⁷ virus/ml. A Blue-violet rabbit weighing 2 kg with its back shaved, was sterilized with 75% ethanol, injected subcutaneously with the virus injection 0.2 ml per site in 100 sites with no leaking, no injecting without the virus injection and no puncturing throughout skin. The injected rabbit was fed for 3 days. When the inflammatory tissue showed that skin surface had visible blains accompanied with changing colour from redness to mauveness and the skin became thick, and subcuticle and hip became swollen, the rabbit was killed by cervical vertebra dislocation and peeled in 15 min. The skin of the rabbit was packed in a plastic bag, preserved at -18°C in refrigerator prior to use. The skin of rabbit weighed 235 g, its SART activity was 0.74 iu/g, and its absorbance was 0.13. The results indicated that it possessed the kallikrein-protease inhibition activity.

### Example 8

### Preparation of rabbit skin

The vaccinia virus Ikeda strain and Japanese white rabbit were used to prepare the subculture antigen according to example 1.

The virus solution of subculture antigen was taken from the -80°C refrigerator, thawed at 30°C in an incubator. 5 ml virus solution was added into 500 ml PBS (-) by a 10-ml syringe, well shaken and diluted to an injection of 10⁹ virus/ml. A Japanese white rabbit weighing 3 kg with its back shaved, was sterilized with 75% ethanol, injected subcutaneously with the virus injection 0.3 ml per site in 200 sites with no leaking, no injecting without the virus injection and no puncturing throughout skin. The injected rabbit was fed for 3 days. When the inflammatory tissue showed that the skin surface had visible blains accompanied with changing colour from redness to mauveness and the skin became thick, and subcuticle and hip became swollen, the rabbit was killed by cervical vertebra dislocation and peeled in 15 min. The skin of the rabbit was packed in plastic bag, preserved at -18°C in a refrigerator prior to use. The skin of the rabbit weighed 335 g, its SART activity was 0.70 iu/g, and its absorbance was 0.12. The results indicated that it possessed the kallikrein-protease inhibition activity.

### Example 9

### Preparation of rabbit skin

The vaccinia virus Dairen strain and Japanese white rabbit were used to prepare for the subculture antigen according to example 1.

The virus solution of the subculture antigen was taken from the -80°C refrigerator, thawed at 30°C in an incubator. 5 ml of the virus solution was added into 500 ml of PBS (-) by a 10-ml syringe, well shaken and diluted to an injection of 10⁶ virus/ml. A Japanese white rabbit weighing 3 kg with its back shaved, was sterilized with 75 % ethanol, injected subcutaneously with the virus injection 0.1 ml per site in 200 sites with no leaking, no injecting without the virus injection and no puncturing throughout the skin. The injected rabbit was fed for 3 days. When the inflammatory tissue showed that the skin surface had visible blains accompanied with changing colour from redness to mauveness and the skin became thick, and subcuticle and hip became swollen, the rabbit was killed by cervical vertebra dislocation and peeled in 15 min. The skin of the rabbit was packed in a plastic bag, preserved at -18°C in a refrigerator prior to use. The skin of rabbit weighed 336 g, its SART activity was 0.61 iu/g, and its absorbance was 0.14. The results indicated that it possessed the kallikrein-protease inhibition activity.

### Example 10

### Preparation of rabbit skin

The vaccinia virus EM-63 strain and Japanese white rabbit were used to prepare the subculture antigen according to example 1.

The virus solution of the subculture antigen was taken from the -80°C refrigerator, thawed at 30°C in incubator. 5 ml of the virus solution was added into 500 ml of PBS(-) by a 10-ml syringe, well shaken and diluted to an injection of 10⁷ virus/ml. A Japanese white rabbit weighing 3 kg with its back shaved, was sterilized with 75% ethanol, injected subcutaneously with the virus injection 0.2 ml per site in 200 sites with no leaking, no injecting without the virus injection and no puncturing throughout skin. The injected rabbit was fed for 3 days. When the inflammatory tissue showed that the skin surface had visible blains accompanied with changing colour from redness to mauveness and the skin became thick, and subcuticle and hip became swollen, the rabbit was killed by cervical vertebra dislocation and peeled in 15 min. The skin of the rabbit was packed in a plastic bag, preserved at -18°C in a refrigerator prior to use. The skin of the rabbit weighed 335 g, its SART activity was 0.66 iu/g, and its absorbance was 0.12. The results indicated that it possessed the kallikrein-protease inhibition activity.

### Example 11

### Extraction of bioactive substances

200 g each of the rabbit skins prepared according to example 1~10 were cut into pieces of 1 cm², added into 4 times (w/w) of 3% phenol solution, placed at 4°C for 72h, centrifuged after the liquid changed into an emulsion. The centrifuged liquid was filtrated to collect a brown solution A. After the solution A's pH was adjusted to 5.0 by 1M HCl, solution A was boiled in a water bath for 40 min and cooled down to 28°C immediately, centrifuged, and filtrated to collect solution B. After pH of the filtrate was adjusted to 9.2 by 1M NaOH, the solution B was boiled for 40 min in a water bath, cooled down to 28°C immediately, and filtrated to collect solution C. After pH of the filtrate was adjusted to 4.5 by 1M HCl, 50 g of activated charcoal was added to the solution C at 30°C, stirred continuously for 4h, stopped stirring, left it for 30min, removed the supernatant, filtrated under nitrogen atmosphere. The activated charcoal was dipped in injectable water and washed, filtrated and removed filtrate to collect and reserve the activated charcoal. The activated charcoal was then added into 400 ml injectable water in which pH was adjusted to 11.0 by 1M NaOH, stirred continuously for 4h, filtrated with a 0.45-µm Millipore filter under nitrogen atmosphere, washed by 40 ml of injectable water to collect solution D. After pH of the solution D was adjusted to 6.0 by 1M HCl, the vessel was sealed, heated up and the temperature was kept at 121°C for 20min and cooled down to under 40°C to collect solution E. The solution E in decompression distillator under nitrogen was decompressed and evaporated at 60°C till the volume was 5 ml and filtrated to collect preparation of 5 ml of a solution containing bioactive substances. The content of amino acids and nucleic acids given below was determined (µg/ml):

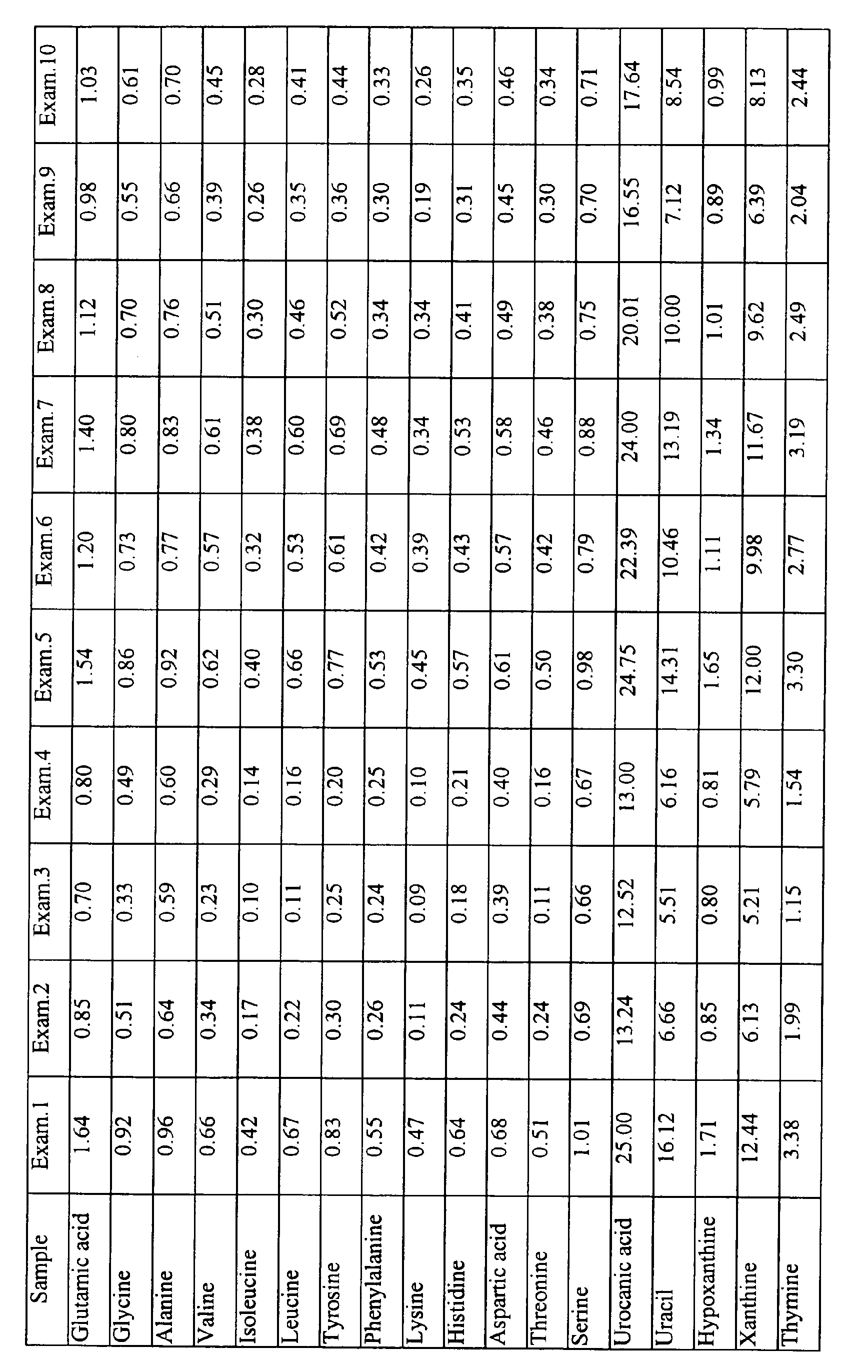

### Example 12

### Preparation of drug

The analgesic injection was prepared by the formula given below using a regular method.

| | |
|---|---|
| Preparation from rabbit skin according to example 2 | 5 ml |
| NaCl | 2.6 g |
| Injectable distilled water | 300 ml |

### Example 13

### Preparation of tablet

The analgesic tablet was prepared by the formula given below using a regular method.

| | |
|---|---|
| Preparation from rabbit skin according to example 1 | 50 ml |
| Lactose | 125 mg |
| Crystalline cellulose | 20 g |
| Magnesium stearic acid | 5 mg |

### Example 14

### Preparation of health food

The health food was prepared by the formula given below using a regular method.

| | |
|---|---|
| Preparation from rabbit skin according to example 1 | 50 ml |
| Sucrose | 125 mg |
| Citric acid | 20 mg |
| Vitamin C | 5 mg |
| Water | 1000 ml |

## Claims

1. A rabbit skin containing biologically active substances, **characterized in that** it is obtained by the following process including vaccinating rabbit skin tissues with vaccinia virus, feeding rabbit vaccinated with vaccinia virus, killing the rabbit when its skin tissues is inflamed enough, and peeling the rabbit.

2. The rabbit skin according to claim 1, wherein said vaccinia virus is vaccinia virus Lister strain.

3. The rabbit skin according to claim 1, wherein said vaccinia virus is vaccinia virus Ikeda strain.

4. The rabbit skin according to claim 1, wherein said vaccinia virus is vaccinia virus Dairen strain.

5. The rabbit skin according to claim 1, wherein said vaccinia virus is vaccinia virus EM-63 strain.

6. The rabbit skin according to claim 1, wherein said vaccinating of rabbit skin tissues with vaccinia virus is effected by injecting subcutaneously 0.1~0.4 ml solution containing 10⁶~10⁹ viruses/ml at each site, 100 to 250 sites per rabbit weighing 1.5~3 kg.

7. The rabbit skin according to claim 1, wherein said rabbit is Japanese white rabbit.

8. The rabbit skin according to claim 1, wherein said rabbit is New Zealand white rabbit.

9. The rabbit skin according to claim 1, wherein the said rabbit is Chinese rabbit.

10. The rabbit skin according to claim 1, wherein the said rabbit is Blue-violet rabbit.

11. The rabbit skin according to claim 1, wherein said killing the rabbit when its skin tissues is inflamed enough is effected when the rabbit skin inflammatory tissue shows visible blains accompanied with changing colour from redness to mauveness and becomes thick, and its subcuticle and hip become swollen.

12. The rabbit skin according to any one of claims 1~11, which possesses 0.5 iu/g SART activity or more.

13. The rabbit skin according to any one of claims 1~11, which possesses the kallikrein-protease inhibition activity.

14. Use of the rabbit skin according to any one of claims 1~13, **characterized in that** the rabbit skin is used for preparing a drug.

15. Use of the rabbit skin according to any one of claims 1~13, **characterized in that** the rabbit skin is used for preparing health food.
